Europäisches Patentamt

**European Patent Office**

Office européen des brevets

(19)

(11) Veröffentlichungsnummer: **0 065 193
B1**

(12)

# EUROPÄISCHE PATENTSCHRIFT

(45) Veröffentlichungstag der Patentschrift:
04.12.85

(51) Int. Cl.⁴: **C 09 B 61/00,** C 09 B 67/42,
A 23 L 1/275, A 61 K 31/00

(21) Anmeldenummer: **82103838.7**

(22) Anmeldetag: **05.05.82**

(54) Verfahren zur Herstellung von feinverteilten, pulverförmigen Carotinoid- bzw. Retinoidpräparaten.

(30) Priorität: **15.05.81 DE 3119383**

(43) Veröffentlichungstag der Anmeldung:
**24.11.82 Patentblatt 82/47**

(45) Bekanntmachung des Hinweises auf die Patenterteilung:
**04.12.85 Patentblatt 85/49**

(84) Benannte Vertragsstaaten:
**AT BE CH DE FR GB IT LI NL**

(56) Entgegenhaltungen:
DE - B - 1 211 911
FR - A - 1 219 213
FR - A - 2 281 961
US - A - 3 125 451

CHEMICAL ABSTRACTS, Band 83, Nr. 11, 24. November
1975, Seite 414, Nr. 176994e, COLUMBUS, OHIO, USA

Die Akte enthält technische Angaben, die nach dem
Eingang der Anmeldung eingereicht wurden und die
nicht in dieser Patentschrift enthalten sind.

(73) Patentinhaber: **BASF Aktiengesellschaft,
Carl-Bosch-Strasse 38, D-6700 Ludwigshafen (DE)**

(72) Erfinder: **Horn, Dieter, Dr., Schroederstrasse 69,
D-6900 Heidelberg (DE)**
Erfinder: **Schmidt, Hans-Wilhelm, Dr.,
Robert-Blum-Strasse 25, D-6800 Mannheim 1 (DE)**
Erfinder: **Ditter, Walter, Feuerbachstrasse 7,
D-6900 Heidelberg (DE)**
Erfinder: **Hartmann, Horst, Lindenstrasse 45,
D-6737 Boehl-Iggelheim (DE)**
Erfinder: **Lueddecke, Erik, Dr.,
Thomas-Mann-Strasse 27, D-6704 Mutterstadt (DE)**
Erfinder: **Schmieder, Klaus, Dr., Sonnenstrasse 12B,
D-6710 Frankenthal (DE)**

## Beschreibung

Die Erfindung betrifft ein Verfahren zur Überführung von Carotinoiden und Retinoiden in feinverteilte, pulverförmige Form, die insbesondere zum Färben von Lebens- und Futtermitteln benötigt wird.

Die Carotinoide bilden eine Gruppe von Farbpigmenten mit gelber bis roter Farbtonnuance, die in der Natur weitverbreitet vorkommen und vielen Nahrungsmitteln eine charakteristische Färbung verleihen. Als wichtigste Vertreter dieser Stoffklasse seien $\beta$-Carotin, $\beta$-Apo-8'-carotinal, Canthaxanthin und Citranaxanthin genannt. Sowohl für die Lebensmittel- und Futtermittelindustrie als auch für die pharmazeutische Technologie stellen diese synthetisch herstellbaren Sbustanzen z. B. als Ersatz für künstliche Farbstoffe wichtige Farbkörper dar oder sind z. B. wegen ihrer Pro-Vitamin-A-Aktivität von Interesse.

Alle Carotinoide sind in Wasser unlöslich, während in Fetten und Ölen eine ebenfalls nur geringe Löslichkeit gefunden wird. Diese begrenzte Löslichkeit sowie die hohe Oxidationsempfindlichkeit behindern eine direkte Anwendung der relativ grobkörnigen bei der Synthese erhaltenen Produkte in der Einfärbung von Lebens- oder Futtermitteln, da nur eine geringe Farbausbeute erzielt werden kann und die Substanzen in grobkristalliner Form nur schlecht resorbiert werden. Diese für die praktische Verwendung der Carotinoide nachteiligen Effekte wirken sich insbesondere im wäßrigen Medium aus, da sie darin gänzlich unlöslich sind.

Zur Verbesserung der Farbausbeuten und zur Erhöhung der Resorbierbarkeit sind verschiedene Verfahren beschrieben worden, die alle das Ziel haben, die Kristallitgröße der Wirkstoffe zu verkleinern und auf einen Teilchengrößenbereich von kleiner 10 μm zu bringen. So kann z. B. nach Chimia 21, 329 (1967), $\beta$-Carotin zusammen mit Speiseöl unter Stickstoffatmosphäre in einer Kolloidmühle bis auf eine Partikelgröße von 2 bis 5 μm vermahlen werden. Gemäß Food. Technol. 12, 527 (1958), bewirkt dabei das umhüllende Öl gleichzeitig einen Oxidationsschutz für den Wirkstoff. Eine so erhaltene Suspension, die bis zu 20 oder 30% Wirkstoff enthält, kann mit Erfolg zur Einfärbung von Fetten und Ölen benutzt werden, da trotz der geringen Löslichkeit diese ausreicht, um die Kristalle bei der üblicherweise angewandten geringen Konzentration in Lösung zu bringen.

Weit schwieriger gestaltet sich dagegen die Konfektionierung der Wirkstoffe für die Anwendung im wäßrigen Medium. Im wäßrigen Medium ist keine Löslichkeit der Carotinoide nachweisbar, und die gewünschte Färbe- und Resorptionseigenschaften können nur über den möglichst feinverteilten kristallinen Zustand erzielt werden. Wünschenswert ist dabei eine Partikelgröße von kleiner 1 μm, die durch Vermahlung entweder überhaupt nicht oder nur unter Schädigung des Wirkstoffes erreichbar ist. Versuche, die Carotinoide unter Verwendung eines wasserlöslichen organischen Lösungsvermittlers, wie Alkohol oder Aceton zunächst zu lösen und dann durch Verdünnen mit Wasser feinkrsitallin auszufällen, scheiterten bisher an der zu geringen Löslichkeit der Carotinoide in diesen Lösungsmitteln. So beträgt z. B. die Löslichkeit von $\beta$-Carotin bei Raumtemperatur in Aceton weniger als 0,1 Gew.-% und in Äthanol weniger als 0,01 Gew.-%.

Andere Verfahren zur Herstellung eines Präparates mit Feinverteilung der Wirkstoffe beruhen auf deren Aufbringen auf Trägermaterialien wie Stärke, Pektin oder Trockenmilchpulver, wobei z. B. eine Lösung des Wirkstoffes in Öl gemäß DE-PS 642 307 oder Chloroform gemäß DE-PS 861 637 und CH-PS 304 023 auf die Trägermaterialien aufgesprüht wird. Die erhaltenen Präparate sind jedoch nicht universell in wäßrigen Medien dispergierbar und genügen nicht den üblichen Anforderungen an die Lagerstabilität, da die an der Oberfläche abgelagerten Wirkstoffe rasch oxidativ zerstört werden. Schließlich sind noch Verfahren zu nennen, bei denen Wirkstoffe in Form ihrer Öllösungen in Kolloide, wie Gelatine, emulsionsartig eingebettet werden, wie dies in Chimia 21, 329 (1967), und in FR-PS 1 056 114 sowie in US-PS 2 650 895 beschrieben ist. Die Wirkstoffkonzentrationen in den so hergestellten Präparaten sind wegen der geringen Öllöslichkeit der Wirkstoffe jedoch gering.

Einen gewissen Fortschritt demgegenüber stellen vorbekannte Verfahren dar, bei denen man den Wirkstoff in einem mit Wasser nicht mischbaren Lösungsmittel, vorzugsweise einem Chlorkolenwasserstoff wie Chloroform oder Methylenchlorid löst, die Lösung durch Homogenisieren in einer Gelatine-Zucker-Lösung emulgiert und aus der Emulsion schließlich das Lösungsmittel abzieht, wobei der Wirkstoff in feinkrsitalliner Form freigesetzt wird. Dieses Verfahren ist in Chimia 21, 329 (1967), sowie in DE-AS 12 11 911 und DE-OS 25 34 091 beschrieben. Aus der erhaltenen Suspension wird dann durch Entwässern ein feinverteiltes Pulver gewonnen.

Dieses Verfahren hat aber den Nachteil, daß chlorierte Kohlenwasserstoffe verwendet werden müssen, um eine ausreichend hohe Wirkstoffkonzentration in der Emulsionsphase zu erzielen. Die vollständige Entfernung der chlorierten Kohlenwasserstoffe, die aus toxikologischen Gründen erforderlich ist, kann aber technisch nur schwer erzielt werden.

Es bestand daher die Aufgabe, ein Verfahren vorzuschlagen, das die genannten Nachteile nicht aufweist und das gestattet, sehr feinverteilte freifließende Pulver der Carotinoide herzustellen.

Diese Aufgabe wurde erfindungsgemäß gelöst mit einem Verfahren zur Herstellung von feinverteilten, pulverförmigen Carotinoidpräparaten, insbesondere zum Färben von Lebens- und Futtermitteln, wobei man ein Carotinoid in einem flüchtigen, mit Wasser mischbaren organischen Lösungsmittel bei Temperaturen zwischen 50°C und 200°C, vorzugsweise 100 und 180°C, gegebenenfalls unter erhöhtem Druck, innerhalb einer Zeit von weniger als 10 Sekunden löst, aus der erhaltenen moleculardispersen

Lösung sofort durch schnelles Mischen mit einer wäßrigen Lösung eines quellbaren Kolloids bei Temperaturen zwischen 0°C und 50°C das Carotinoid in kolloiddisperser Form ausfällt und die erhaltene Dispersion in an sich bekannter Weise von dem Lösungsmittel und dem Dispergiermedium befreit.

Obgleich die Herstellung der Carotinoidpräparate der bevorzugte Gegenstand der Erfindung ist, wurde ferner gefunden, daß gleichermaßen Retinoide in feinverteilte Form überführt werden können.

Unter Retinoiden wird dabei vor allem all-trans-Retinsäure, 13-cis-Retinsäure und die Ester und Amide dieser Säuren verstanden. Im einzelnen sind die Formeln dieser Retinoide durch D. L. Newton, W. R. Henderson und M. B. Sporn in Cancer Research 40, 3413—3425, beschrieben, so daß auf diese Literaturstelle Bezug genommen wird.

Die erfindungsgemäße Arbeitsweise macht sich den Umstand zunutze, daß die in der Kälte sehr geringe Löslichkeit der Carotinoide in den wassermischbaren Lösungsmitteln mit steigender Temperatur deutlich zunimmt. Jedoch bestand gegen das Erhitzen von Carotinoiden ein starkes Vorurteil, da es in der Regel zu einer teilweisen Zersetzung und zu einer den Farbton beeinflussenden Isomerisierung der Verbindungen führt.

Die Carotinoide, die bei der Durchführung der Erfindung eingesetzt werden können, sind die bekannten, zugänglichen, natürlichen oder synthetischen Vertreter dieser Klasse von Verbindungen, die als farbgebende Mittel brauchbar sind, z. B. Carotin, Lycopin, Bixin, Zeaxanthin, Cryptoxanthin, Citranaxanthin, Lutein, Canthaxanthin, Astaxanthin, $\beta$-Apo-4'-Carotinal, $\beta$-Apo-8'-carotinal, $\beta$-Apo-12'-carotinal, $\beta$-Apo-8'-carotinsäure sowie Ester von hydroxy- und carboxyhaltigen Vertretern dieser Gruppe, z. B. die niederen Alkylester und vorzugsweise die Methyl- und Äthylester. Besonders bevorzugt werden die bisher technisch gut zugänglichen Vertreter wie $\beta$-Carotin, Canthaxanthin, $\beta$-Apo-8'-carotinal und $\beta$-Apo-8'-Carotinsäureester.

Zur Durchführung des erfindungsgemäßen Verfahrens sind vor allem wassermischbare, thermisch stabile, flüchtige, nur Kohlenstoff, Wasserstoff und Sauerstoff enthaltende Lösungsmittel wie Alkohole, Ether, Ester, Ketone und Acetale geeignet. Vorzugsweise werden Ethanol, n-Propanol, Isopropanol, 1,2-Butandiol-1-methylether, 1,2-Propandiol-1-n-propylether oder Aceton verwendet.

Allgemein verwendet man zweckmäßig solche Lösungsmittel, die mindestens zu 10% wassermischbar sind, einen Siedepunkt unter 200°C aufweisen und/oder weniger als 10 Kohlenstoffatome haben.

Als quellbare Kolloide werden beispielsweise Gelatine, Stärke, Dextrin, Pektin, Gummiarabicum, Kasein, Kaseinat oder Mischungen davon verwendet. Es können aber auch Polyvinylakohol, Polyvinylpyrrolidon, Methylcellulose, Carboxymethylcellulose, Hydroxypropylcellulose und Alginate eingesetzt werden. Bezüglich näherer Einzelheiten wird auf R. A. Morton, Fast Soluble Vitamins, Intern. Encyclopedia of Food and Nutrition, Bd. 9, Pergamon Press 1970, S. 128—131, verwiesen. Zur Erhöhung der mechanischen Stabilität des Endproduktes ist es zweckmäßig, dem Kolloid einen Weichmacher zuzusetzen, wie Zucker oder Zuckeralkohole, z. B. Saccharose, Glukose, Lactose, Invertzucker, Sorbit, Mannit oder Glycerin.

Das Verhältnis Kolloid und Weichmacher zu Carotinoidlösung wird im allgemeinen so gewählt, daß ein Endprodukt erhalten wird, das zwischen 0,5 und 20 Gew.-%, vorzugsweise 10%, Carotinoid oder Retinoid, 10 bis 50 Gew.-% eines quellbaren Kolloids, 20 bis 70% eines Weichmachers, alle Prozentangaben bezogen auf die Trockenmasse des Pulvers, sowie gegebenenfalls untergeordnete Mengen eines Stabilisators enthält, wobei das Pulver eine mittlere Teilchengröße des Carotinoids oder Retinoids von weniger als 0,3 μm und eine Halbwertsbreite der Korngrößenverteilung von weniger als 50% und praktisch keinen Anteilen mit einer Korngröße über 1 μm aufweist.

Zur Erhöhung der Stabilität des Wirkstoffes gegen oxidativen Abbau ist es vorteilhaft, Stabilisatoren wie $\alpha$-Tocopherol, t-Butyl-hydroxy-toluol, t-Butylhydroxyanisol oder Ethoxyquine zuzusetzen. Sie können entweder der wäßrigen oder der Lösungsmittel-Phase zugesetzt werden, vorzugsweise werden sie jedoch gemeinsam mit den Farbstoffen und den tensidartigen Stabilisatoren in der Lösungsmittel-Phase gelöst. Unter Umständen kann es auch vorteilhaft sein, zusätzlich in der Lösungsmittelphase ein Öl oder einen Fettstoff zu lösen, der dann gemeinsam mit den Wirkstoffen und den genannten Zusatzstoffen beim Mischen mit der wäßrigen Phase extrem feinteilig ausgefällt wird.

Die nach Ausfällung der Carotinoide erhaltene Dispersion kann noch zur Erhöhung der Wirkstoffkonzentration aufkonzentriert werden, indem man das kolloiddisperse System entweder durch Zusatz von Salz oder durch Einstellung eines geeigneten pH-Wertes ausflockt und damit in eine Form überführt, aus der durch Filtrieren oder Zentrifugieren auf einfache Weise ein Teil des Dispergiermediums abgetrennt werden kann, wobei die feinverteilten Carotinoide in der flüssigen Phase verbleiben. Besonders vorteilhaft läßt sich die durch den pH-Wert steuerbare Bildung eines abfiltrierbaren bzw. sedimentierbaren Koazervats bei Verwendung einer Mischung von Gelatine und Gummiarabicum als quellbares Kolloid zur Erhöhung der Feststoffkonzentration in der Dispersion nutzen.

Je nach Art und Menge des verwendeten Kolloids erhält man eine tiefgefärbte viskose Flüssigkeit, die im Falle eines gelierfähigen Kolloids gelartig erstarrt. Die Entfernung des Lösungsmittels kann je nach Siedepunkt in an sich bekannter Weise, z. B. durch Destillation, gegebenenfalls unter vermindertem Druck, oder durch Extraktion mit einem mit Wasser nicht mischbaren Lösungsmittel, erfolgen. In diesem Falle hat es sich als zweckmäßig und möglich erwiesen, das bei Verwendung von Isopropanol erhaltene Azeotrop ohne Wasserentfernung unmittelbar als Lösungsmittel einzusetzen. Vorzugsweise erfolgt sie jedoch gemeinsam mit der Entfernung des Wassers durch Sprühtrocknung oder Sprühgranulation.

Man erhält ein Trockenpulver, das bei Verwendung eines wasserlöslichen Kolloids erneut in Wasser unter Erzielung einer gleichmäßigen Feinverteilung des Wirkstoffes im Korngrößenbereich <1 µm gelöst werden kann. Im photochemischen Stabilitätstest erweist sich das so erhaltene Wirkstoff-Hydrosol trotz der Feinverteilung als außerordentlich stabil.

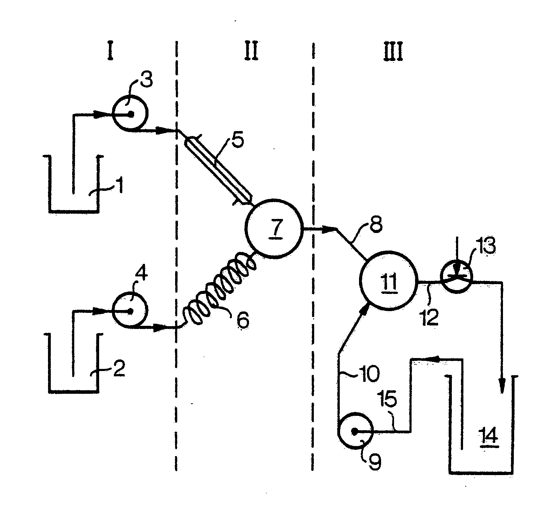

Im einzelnen führt man das erfindungsgemäße Verfahren beispielsweise mit einer Apparatur, wie sie in Fig. 1 schematisch dargetellt ist, wei folgt durch:

Die Apparatur gliedert sich in die Teile I, II und III. Teil II ist der Hochtemperaturabschnitt, während in den Teilen I und III die Temperaturen weniger als 50° C betragen.

Im Gefäß (1) wird eine Suspension des Carotinoids in dem ausgewählten Lösungsmittel in Konzentrationen von 2 bis 40 Gew.-%, bezogen auf die Mischung, gegebenenfalls unter Zusatz von 0,1 bis 10 Gew.-% an Stabilisatoren, vorgelegt. Gefäß (2) enthält das Lösungsmittel ohne Beimischung des Carotinoids. Über die Pumpen (3) bzw. (4) werden die Wirkstoff-Suspension und das Lösungsmittel der Mischkammer (7) zugeführt, wobei das Mischungsverhältnis durch Wahl der jeweiligen Förderleistung der Pumpen vorgegeben werden kann und so gewählt wird, daß je nach Lösungsmittel und Verweilzeit eine Carotinoid-Konzentration in der Mischkammer von 0,5 bis 10 Gew.-%, bezogen auf die Lösung, entsteht. Das Mischkammervolumen (7) ist so bemessen, daß bei der gewählten Förderleistung der Pumpen (3) und (4) die Verweilzeit in (7) vorzugsweise weniger als 1 Sekunde beträgt.

Das Lösungsmittel wird vor Eintritt in die Mischkammer über den Wärmetauscher (6) auf die gewünschte Temperatur gebracht, während die Wirkstoffsuspension durch Zuführung über die thermisch isolierte Zuleitung (5) bei Temperaturen unterhalb 50° C gehalten wird. Durch turbulente Mischung in (7) erfolgt im Temperaturbereich 50 bis 200° C, vor allem 100 bis 180° C, vorzugsweise jedoch bei 140 bis 180° C, die Lösung des Wirkstoffes und die erhaltene Lösung tritt über (8) nach kurzer Verweilzeit, vorzugsweise von weniger als einer Sekunde, in die zweite Mischkammer (11) ein, in der durch Zumischen von Wasser oder einer wäßrigen Schutzkolloidlösung über die Pumpe (9) und die Zuleitung (10) die Ausfällung des Wirkstoffes in kolloiddisperser Form erfolgt. Über Leitung (12) wird sodann die feinteilige Wirkstoffdispersion über das Überdruckventil (13) ausgetragen und dem Vorratsgefäß (14) zugeführt. Zur Erzielung einer möglichst hohen Wirkstoffkonzentration kann die Dispersion über die Saugleitung (15) im Kreis geführt werden.

Bei Einstellung des Überdruckventils (13) auf Drücke oberhalb 1 bar können in dem neuen Verfahren sogar Lösungsmittel bei Temperaturen oberhalb ihres Siedepunktes (bei Normaldruck) verwendet werden.

Aus der Dispersion kann ein pulverförmiges Präparat in an sich bekannter Weise, z. B. gemäß den Angaben der DE-OS 2 534 091 durch Sprühtrocknen oder durch Sprühkühlen oder durch Einhüllen der Teilchen, Abtrennen und Trocknen im Wribelbett erfolgen.

Zum Sprühtrocknen wird die Dispersion entweder zuerst vom Lösungsmittel durch Destillation, vorzugsweise unter vermindertem Druck, oder durch Extraktion mit einem mit Wasser nicht mischbaren Lösungsmittel befreit oder die gesamte Mischung wird sprühgetrocknet und so Wasser und Lösungsmittel zusammen im Sprühturm abgezogen.

Am Boden des Sprühturms fällt das Carotinoidpulver entweder bereits trocken und rieselfähig an. In manchen Fällen kann es zweckmäßig sein, die vollständige Trocknung zusätzlich in einem Wirbelbett vorzunehmen.

Anstelle der Herstellung der Pulverzubereitung durch Sprühtrocknen können auch beliebige andere Methoden angewendet werden, um die in der Wasser/Lösungsmittel-Dispersion bereits feinverteilten Carotinoide in die Pulverform zu überführen.

Ein bekanntes und hier gleichermäßen anwendbares Verfahren besteht z. B. darin, die vom Lösungsmittel befreite Dispersion mit Paraffinöl zu emulgieren, die Mischung abzukühlen, das Paraffinöl von den eingehüllten Carotinoidteilchen zu trennen, das erhaltene Carotinoidpräparat mit Benzin zu waschen und im Wirbelbett zu trocknen.

Bei der erfindungsgemäßen Arbeitsweise ist besonders überraschend, daß mit den genannten wassermischbaren Lösungsmitteln bei erhöhter Tempratur trotz der geringen Kontaktzeit von weniger als 1 Sekunde die Lösegeschwindigkeit ausreicht, um molekulardisperse Lösungen der Wirkstoffe mit einem Gehalt von 0,5 bis 10% herzustellen, wie dies bisher nur mit Halogenkohlenwasserstoffen möglich war, und daß trotz der hohen Temperaturen die Isomerisierungsgeschwindigkeit nicht ausreicht, um innerhalb der kurzen Verweilzeit im Lösungszustand einen meßbaren Anstieg der Konzentration von cis-Isomeren zu verursachen.

Es war weiterhin überraschend, daß beim Mischen der Carotinoidlösung, die gegebenenfalls noch Stabilisatoren wie Ascorbylpalmitat, Mono- und Diglyceride, Ester von Monoglyceriden mit Essigsäure, Zitronensäure, Milchsäure oder Diacetylweinsäure, Polyglycerinfettsäureester, Sorbitanfettsäureester, Propylenglykol-Fettsäureester, Stearoyl-2-Lactylate oder Lecithin enthalten kann, mit der wäßrigen Lösung der quellbaren Kolloide ein außerordentlich feinteiliges und dennoch sehr stabiles Carotinoidpräparat erhalten wird, wobei außerdem noch der Feinheitsgrad durch die Wahl der der Carotinoidlösung zugesetzten Stabilisatoren gesteuert werden kann. Dieser Feinverteilungszustand des Wirkstoffes bleibt auch während der Entfernung des flüchtigen Lösungsmittels, z. B. durch Sprühtrocknen, erhalten. Es ist ohne Schwierigkeit möglich, Präparate zu erhalten, in denen der Hauptanteil des Wirkstoffes in einer Korngröße von 0,2 µm vorliegt, ohne daß gleichzeitig Wirkstoffteilchen von über 1 µm vorhanden sind.

In den nachfolgenden Beispielen wird die Durchführung des erfindungsgemäßen Verfahrens näher erläutert.

### Beispiel 1

5 g $\beta$-trans-Carotin werden in 80 g einer Lösung von 0,8 g dl-$\alpha$-Tocopherol und 0,8Ascorbylpalmitat in 1,2-Butandiol-1-methylether bei 25°C suspendiert und bei einer Mischungstemperatur von 135° —140°C mit 100 g 1,2-Butandiol-1-methylether (Sp. 138°C) in der Mischkammer (7) (Fig. 1) bei einer Verweildauer von 0,3 Sekunden gemischt, und die dabei entstehende molekulardisperse Lösung unmittelbar anschließend über die Zuleitung (8) der Mischkammer (11) zugeführt, in der durch Vermischen mit 800 g einer mit 1 n NaOH auf pH 9,5 eingestellten wäßrigen Gelatinelösung, die neben 13,2 g Gelatine, 6 g Dextrose und 3,6 g Dextrin enthält, das $\beta$-Carotin, in kolloiddisperser Form ausgefällt. Der gesamte Prozeß erfolgt unter Einstellung des Druckbegrenzungsventils (13) auf 5 bar, um eine Verdampfung des Lösungsmittels während der Feinverteilung zu vermeiden.

Im Auffanggefäß (14) wird eine kolloid-disperse $\beta$ Carotin-Dispersion mit orange-gelber Farbtonnuance erhalten.

Durch Laser-Photonen-Korrelationsspektroskopie nach B. Chu., Laser Light Scattering; Academic Press, New York, 1974, wurde die Teilchengröße des $\beta$-Carotins zu 0,25 µm bei einer Verteilungsbreite von ±50% bestimmt.

Die spektroskopische Analyse des $\beta$-Carotins gemäß FAO Nutrition Meetings Report Series No. 54 B,. WHO/Food Add./7, 18th Report, 1974, lieferte bei den Wellenlängen 455 und 340 nm ein Extinktionsverhältnis von $E_{455}/E_{340} = 15,3$ und erfüllt damit die Spezifikation für all-trans-$\beta$-Carotin.

Durch Sprühtrocknung der Dispersion wird ein freifließendes Trockenpulver erhalten, das sich in Wasser unter Bildung einer klaren gelbstichigen Dispersion löst.

### Beispsiel 2

Wie in Beispiel 1 beschrieben, wird eine molekulardisperse Lösung von 5 g trans-$\beta$-Carotin in 1,2-Butandiol-1-methylether in der Mischkammer hergestellt und unmittelbar anschließend der Mischkammer (11) zugeführt, in der durch Vermischen mit 800 g einer mit 1 n NaOH auf pH 9,5 eingestellten wäßrigen Lösung von 7,9 g Gelatine und 5,3 g Gummiarabicum sowie 6 g Dextrose und 3,6 g Dextrin das $\beta$-Carotin in kolloid-disperser Form ausgefällt wird. Der pH-Wert der orange-gelben Dispersion wird sodann mit 1 n Schwefelsäure auf 4 bis 4,5 eingestellt und dadurch der Feststoffanteil der Dispersion ausgeflockt. Durch Abtrennen der flüssigen Phase und mehrmaliges Waschen wird ein Produkt erhalten, das frei von Lösungsmittelresten ist und durch Sprühtrocknung oder Sprühgranulation in ein Trockenpulver überführt werden kann.

### Beispiel 3

Wie in Beispiel 1 angegeben, jedoch unter Verwendung von Aceton als Lösungsmittel und bei Einstellung des Druckbegrenzungsventils (13) auf 25 bar werden 5 g trans-$\beta$-Carotin in 85 g Aceton gemeinsam mit 0,8 g dl-$\alpha$-Tocopherol und 0,8 g Ascorbylpalmitat suspendiert und mit 130 g Aceton in Mischkammer (7) kontinuierlich gemischt. Bei einer Dosierleistung von 2 l/h auf der Suspensionsseite und 3 l/h auf der Lösungsmittelseite beträgt die Verweilzeit in der Mischzelle 0,35 Sekunden. Die molekulardisperse Lösung wird sodann in Mischkammer (11) mit einer auf pH 9,5 eingestellten wäßrigen Lösung von 13,2 g Gelatine, 5,9 g Dextrose, 1,8 g Dextrin und 1,8 g eines Diacetylweinsäureesters eines Fettsäure-monoglycerids bei einer Durchsatzgeschwindigkeit von 45 l/h gemischt. Es wird eine kolloid-disperse Wirkstoffsuspension mit orange-gelber Farbton-Nuance erhalten. Die Teilchengrößenanalyse lieferte einen Mittelwert von 0,27 µm bei einer Verteilungsbreite von ±46%. Die spektroskopische Analyse lieferte ein spezifikationsgerechtes Extinktionsverhältnis von $E_{455}/E_{340} = 15,2$.

Nach Abtrennen des Lösungsmittels unter vermindertem Druck bei 50°C in einer Destillationsapparatur wird eine kolloid-disperse Wirkstoffdispersion erhalten, die durch Sprühtrocknung in ein stabiles, leicht wasserlösliches Trockenpulver überführt werden kann.

### Beispiele 4—6

Entsprechend der Arbeitsweise von Beispiel 3 werden die in Tabelle I aufgeführten wasserdispergierbaren, sprühgetrockneten $\beta$-Carotinpulver der angegebenen Zusammensetzungen erhalten, mit einem Gehalt von 4%, 6% und 10% $\beta$-Carotin.

Tabelle I

|  | 4% | 6% | 10% |
|---|---|---|---|
| $\beta$-Carotin | 1 g | 1,5 g | 2,7 g |
| **Lösungsmittelphase** | | | |
| dl-$\alpha$-Tocopherol | 0,8 g | 0,8 g | 0,8 g |
| Ascorbylpalmitat | 0,8 g | 0,8 g | 0,8 g |
| **Wäßrige Phase** | | | |
| Dextrose | 5,9 g | 5,9 g | 5,9 g |
| Dextrin | 1,8 g | 1,8 g | 1,8 g |
| Zitronensäureester eines Fettsäuremonoglycerids | 1,8 g | 1,8 g | 1,8 g |
| Gelatine | 13,2 g | 13,2 g | 13,2 g |

Beispiele 7—9

Wie in Beispiel 3 angegeben, werden von den Carotinoiden Canthaxanthin, Citranaxanthin und $\beta$-Apo-8'-Carotinsäureethylester Trockenpulver der in Tabelle II angegebenen Zusammenstellung hergestellt, die jeweils 11,6% Carotinoid enthalten.

Tabelle II

|  | Beispiel 7 | Beispiel 8 | Beispiel 9 |
|---|---|---|---|
| Carotinoid | 5 g Canthaxanthin | 5 g Citranaxanthin | 5 g $\beta$-Apo-8'-Carotinsäureethylester |
| **Lösungsmittelphase** | | | |
| dl-$\alpha$-Tocopherol | 0,8 g | 0,8 g | 0,8 g |
| Ascorbylpalmitat | 0,8 g | 0,8 g | 0,8 g |
| **Wäßrige Phase** | | | |
| Dextrose | 5,9 g | 5,9 g | 5,9 g |
| Dextrin | 3,6 g | 3,6 g | 3,6 g |
| Gelatine | 27,1 g | 27,1 g | 27,1 g |
| Teilchengröße | 0,15 ± 30% μm | 0,19 ± 37% μm | 0,23 ± 40% μm |

Beispiele 10—13

Wie in den Beispielen 7 bis 9 angegeben, jedoch unter Verwendung von Ethanol als Lösungsmittel, werden von den Carotinoiden $\beta$-Carotin, Canthaxanthin, Citranaxanthin und $\beta$-Apo-8'-Carotinsäureet-

**0 065 193**

hylester Trockenpulver hergestellt. Die Größenverteilung der Carotinoidteilchen in den Präparaten ist in Tabelle III zusammengestellt.

Tabelle III

| Carotinoid | Mittlere Teilchengröße | Verteilungsbreite |
|---|---|---|
| $\beta$-Carotin | 0,18 µm | ±42% |
| Canthaxanthin | 0,14 µm | ±27% |
| Citranaxanthin | 0,16 µm | ±16% |
| $\beta$-Apo-8'-Carotinsäureethylester | 0,18 µm | ±22% |

### Beispiel 14

30 g Canthaxanthin werden in 240 g Isopropanol gemeinsam mit 1,1 g Ascorbylpalmitat und 6,4 g Ethoxyquin suspendiert und bei Einstellung des Druckbegrenzungsventils (13) auf 30 bar mit 370 g Isopropanol in Mischkammer (7) kontinuierlich gemischt. Bei einer Dosiergeschwindigkeit von 6 l/h auf der Suspensionsseite und von 9 l/h auf der Lösungsmittelseite wird in der Mischkammer (7) eine Mischungstemperatur von 173°C eingestellt. Nach einer Verweilzeit von 0,3 Sekunden wird die molekular-disperse Lösung in Mischkammer (11) mit einer auf pH 9,5 eingestellten Lösung von 38,6 g Gelatine und 105 g Dextrose in 4000 g Wasser bei einer Durchsatzgeschwindigkeit von 80 l/h Isobutanol gemischt. Man erhält eine kolloid-disperse Wirkstoffsuspension mit roter Farbton-Nuance. Die Teilchengrößenanalyse liefert einen Mittelwert von 0,15 µm bei einer Verteilungsbreite von ±31%.

Nach Abtrennen des Lösungsmittels unter vermindertem Druck in einer Destillationsapparatur wird eine Wirkstoff-Dispersion erhalten, die durch Sprühtrocknung in ein stabiles, wasserlösliches Trockenpulver überführt werden kann. Nach Auflösen in Wasser wird ein Teilchengröße von 0,17 µm ±30% gemessen.

### Beispiel 15

Verfährt man, wie in Beispiel 14 angegeben, verwendet jedoch die azeotrope Isopropanol/Wassermischung mit 12 Gew.-% Wasser, so gelingt in analoger Weise die Feinverteilung von Canthaxanthin. Es wird eine Wirkstoffsuspension erhalten, die durch eine mittlere Teilchengröße vn 0,16 µm ±35% gekennzeichnet ist.

### Beispiel 16

5 g 13-Z-Vitamin-A-säure werden in 40 g Isopropanol suspendiert und mit einer Dosiergeschwindigkeit von 3 l/h mit 46 g Isopropanol bei einer Dosiergeschwindigkeit von 4,5 l/h in Mischkammer (7) kontinuierlich gemischt. Die stationäre Mischtemperatur beträgt 172°C. In Mischkammer (11) wird unmittelbar anschließend durch turbulentes Mischen mit einer auf pH 3,0 eingestellten Lösung von 10 g Gelatine und 26 g Dextrose in 1000 g Wasser bei einer Dosiergeschwindigkeit von 80 l/h der Wirkstoff kontinuierlich ausgefällt. Man erhält eine kolloid-disperse Wirkstoffsuspension von gelber Farbton-Nuance. Die Teilchengröße beträgt 0,23 µm ±25%. Nach Abtrennen des Lösungsmittels liefert die Sprühtrocknung ein wasserlösliches Trockenpulver.

### Beispiel 17

Man verfährt wie in Beispiel 15, verwendet jedoch N-4-Hydroxyphenylretinamid als Wirkstoff und eine Mischung aus 50 Gew.-% Isopropanol und 50 Gew.-% Wasser als Suspensionsmedium für den Wirkstoff und als Lösungsmittel und löst kontinuierlich 5 g des Retinoids in Mischkammer (7) bei einer Mischungstemperatur von 150°C, mischt kontinuierlich mit 1240 g einer 12 g Gelatine und 33 g Dextrose enthaltenden wäßrigen Lösung in Mischkammer (11) und führt so das Retinoid in eine feindisperse Form über. Die Messung der Teilchengrößenverteilung liefert einen Mittelwert von 0,23 µm und eine Verteilungsbreite von ±44%. Das feinverteilte Produkt wird anschließend nach Abzug des Lösungsmittels durch Sprühtrocknung in ein wasserlösliches Trockenpulver überführt.

7

## Patentansprüche

1. Verfahren zur Herstellung von feinverteilten, pulverförmigen Carotinoid- bzw. Retinoidpräparaten, in denen das Carotinoid bzw. Retinoid im wesentlichen eine Teilchengröße von weniger als 0,5 Mikron besitzen, dadurch gekennzeichnet, daß man ein Carotinoid bzw. das Retinoid in einem flüchtigen, mit Wasser mischbaren, organischen Lösungsmittel bei Temperaturen zwischen 50°C und 200°C, gegebenenfalls unter erhöhtem Druck, innerhalb einer Zeit von weniger als 10 Sekunden löst, aus der erhaltenen molekulardispersen Lösung sofort durch schnelles Mischen mit einer wäßrigen Lösung eines quellbaren Kolloids bei Temperaturen zwischen 0°C und 50°C das Carotinoid in kolloid-disperser Form ausfällt und die erhaltene Dispersion in an sich bekannter Weise von dem Lösungsmittel und dem Dispergiermedium befreit.

2. Verfahren zur Herstellung von feinverteilten, pulverförmigen Carotinoid- bzw. Retinoidpräparaten gemäß Anspruch 1, dadurch gekennzeichnet, daß als wassermischbare flüchtige Lösungsmittel Alkohole, Ketone, Ester, Acetale oder Ether verwendet werden.

3. Verfahren zur Herstellung von Carotinoid- bzw. Retinoidpräparaten gemäß Anspruch 2, dadurch gekennzeichnet, daß als wassermischbare flüchtige Lösungsmittel Aceton, 1,2-Butandiol-1-methylether, 1,2-Propandiol-1-n-propylether, Ethanol, n-Propanol, Isopropanol oder deren Gemische verwendet werden.

4. Verfahren gemäß Ansprüchen 1 bis 3, dadurch gekennzeichnet, daß die Herstellung der molekulardispersen Carotinoid- bzw. Retinoidlösung und die Ausfällung des Carotinoids bzw. Retinoids in feinstdisperser Form kontinuierlich in zwei in Reihe geschalteten Mischkammern erfolgt.

5. Vorrichtung zur Ausführung des Verfahrens gemäß Ansprüchen 1 bis 4, gekennzeichnet durch die Elemente

a1) Vorratsgefäß für eine Carotinoid- bzw. Retinoiddispersion und damit verbundene
a2) Dosiervorrichtung zur ersten Mischkammer,
b1) Vorratsgefäß für das wassermischbare Lösungsmittel mit
b2) Dosiervorrichtung und beheizbarer Zuleitung zur ersten Mischkammer,
c) beheizbare erste Mischkammer in vorzugsweise zylindrischer Form sowie
d) eine damit verbundene weitere Mischkammer, die eine turbulente Mischung der aus der ersten Mischkammer eintretenden Lösung mit der wäßrigen Lösung erlaubt.

6. Pulverförmige Carotinoid- und Retinoidpräparate, hergestellt nach dem Verfahren des Anspruchs 1, enthaltend 0,5 bis 20 Gew.-% eines Carotinoids oder Retinoids, 10 bis 50 Gew.-% eines quellbaren Kolloids, 20 bis 70 Gew.-% eines Weichmachers, alle Prozentangaben bezogen auf die Trockenmasse des Pulvers, sowie gegebenenfalls untergeordnete Mengen eines Stabilisators, gekennzeichnet durch Freiheit von Chlorkolenwasserstoffen, eine mittlere Teilchengröße des Carotinoids oder Retinoids von weniger als 0,3 µm und eine Halbwertsbreite der Korngrößenverteilung von weniger als 50% und praktisch keinen Anteilen mit einer Korngröße über 1 µm.

## Claims

1. A process for the preparation of a finely divided, pulverulent carotinoid or retinoid composition, in which the carotinoid or retinoid essentially has a particle size of less than 0.5 micron, wherein a carotinoid or retinoid is dissolved in a volatile, water-miscible, organic solvent at from 50°C to 200°C, if necessary under superatomospheric pressure, within the space of less than 10 seconds, the carotinoid is immediately precipitated, in a colloidaly disperse form, from the molecularly disperse solution by rapidly mixing the latter with an aqueous solution of a swellable colloid at from 0°C to 50°C, and the resulting dispersion is freed from the solvent and the dispersing medium in a conventional manner.

2. A process for the preparation of a finely divided, pulverulent carotinoid or retinoid composition als claimed in claim 1, wherein the water-miscible volatile solvent used is an alcohol, ketone, ester, acetal or ether.

3. A process for the preparation of a carotinoid or retinoid composition as claimed in claim 2, wherein the water-miscible volatile solvent used is acetone, butane-1,2-diol 1-methyl ether, propane-1,2-diol 1-n-propyl ether, ethanol, n-propanol, isopropanol or mixtures of these.

4. A process as claimed in claims 1 to 3, wherein the preparation of the molecularly disperse carotinoid or retinoid solution and the precipitation of the carotinoid or retinoid in a very finely disperse form are effected continuously in two mixing chambers connected in series.

5. An apparatus for carrying out a process as claimed in claims 1 to 4, comprising

a1) a stock vessel for a carotinoid or retinoid dispersion and, connected therewith,
a2) a metering device leading to the first mixing chamber,
b1) a stock vessel for the water-miscible solvent together with
b2) a metering device and a heatable feed line leading to the first mixing chamber,

c) a heatable first mixing chamber, preferably of cylindrical shape, and
d) a further mixing chamber, connected therewith, wherein the solution entering from the first mixing chamber can be turbulently mixed with the aqueous solution.

6. A pulverulent carotinoid or retinoid composition prepared by a process as claimed in claim 1 and containing from 0.5 to 20% by weight of a carotinoid or retinoid, from 10 to 50% by weight of a swellable colloid and from 20 to 70% by weight of a plasticizer, all percentages being based on the dry weight of the powder, with or without minor amounts of a stabilizer, characterized by freedom from chlorinated hydrocarbons, a mean particle size of the carotinoid or retinoid of less than 0.3 μm and a half-width of the particle size distribution of less than 50%, with virtually no material having a particle size greater than 1 μm.

**Revendications**

1. Procédé de production de préparations de caroténoïdes ou de rétinoïdes pulvérulentes finement divisées, dans lesquelles le caroténoïde ou le rétinoïde possède essentiellement une granulométrie inférieure à 0,5 micron, caractérisé en ce que l'on dissout un caroténoïde ou un rétinoïde en l'espace de moins de dix secondes, à des températures de 50 à 200°C, le cas échéant sous pression accrue, dans un solvant organique volatil, miscible à l'eau, dans la solution à dispersion moléculaire obtenue, on provoque, par un mélange rapide à une température comprise entre 0° et 50°C avec une solution aqueuse d'un colloïde gonflable, la précipitation immédiate du caroténoïde en dispersion colloïdale et on débarrasse la dispersion obtenue d'une manière connue en soi du solvant et de l'agent de dispersion.

2. Procédé de production de préparations de caroténoïdes ou de rétinoïdes pulvérulentes finement divisées suivant la revendication 1, caractérisé en ce que le solvant volatil miscible à l'eau est choisi parmi les alcools, les cétones, les esters, les acétals et les éthers.

3. Procédé de production de préparations de caroténoïdes ou de rétinoïdes suivant la revendication, caractérisé en ce que le solvant volatil miscible à l'eau est choisi parmi l'acétone, l'éther 1-méthylique du butane-diol-1,2, l'éther 2-n-propylique du propane-diol-1,2, l'éthanol, le n-propanol et l'isopropanol et des mélanges de ceux-ci.

4. Procédé suivant les revendications 1 à 3, caractérisé en ce que la préparation de la solution du caroténoïde ou du rétinoïde en dispersion moléculaire et le précipitation du caroténoïde ou rétinoïde à l'état très finement dispersé s'effectuent en continu dans deux chambres de mélange montées en série.

5. Installation pour la mise en oeuvre du procédé suivant les revendications 1 à 4, caractérisée en ce qu'elle comprend les éléments ci-après:

a1) un réservoir pour une dispersion de caroténoïde ou de rétinoïde, combiné avec
a2) un dispositif de dosage pour la première chambre de mélange;
b1) un réservoir pour le solvant miscible à l'eau, combiné avec
b2) un dispositif de dosage et un conduit pouvant être chauffé vers la première chambre de mélange;
c) une première chambre de mélange pouvant être chaufée, de préférence de forme cylindrique, et
d) une deuxième chambre de mélange, reliée à la première, permettant un mélange turbulent de la solution provenant de la première chambre de mélange et de la solution aqueuse.

6. Préparations pulvérulentes de caroténoïde ou de rétinoïde, préparées par le procédé selon la revendication 1, contenant 0,5 à 20% en poids d'un caroténoïde ou d'un rétinoïde, 10 à 50% en poids d'un colloïde gonflable et 20 à 70% en poids d'un plastifiant — tous les pourcentages se rapportant à la matière sèche de la poudre —, ainsi qu'éventuellement une proportion mineure d'un stabilisant, caractérisées en ce qu'elles sont exemptes d'hydrocarbures chlorés et le caroténoïde ou rétinoïde possède une granulométrie moyenne inférieure à 0,3 μ, la largeur de valeur moyenne de la répartition granulométrique étant inférieure à 50%, et sont pratiquement exemptes de particules d'une dimension supérieure à 1 μ.